# EUROPEAN PATENT APPLICATION

(11) **EP 4 530 628 A1**
(43) Date of publication of application: **02.04.2025**
(21) Application number: 22943639.9
(22) Date of filing: 23.05.2022
(51) Int. Cl.: G01N 33/48

(54) **CLASSIFICATION DEVICE, LEARNING DEVICE, CLASSIFICATION METHOD, LEARNING METHOD, AND PROGRAM**

(71) Applicant: NEC Corporation, 108-8001 Tokyo (JP)
(72) Inventor: OMI, Yasuo, Tokyo 108-8001 (JP)
(74) Representative: Betten & Resch
(86) International application number: PCT/JP2022/021049
(87) International publication number: WO 2023/228230

(57) **Abstract**

In order to improve accuracy in classification of a specimen cell between benignancy and malignancy in pathological diagnosis, a classification apparatus (1) includes: an acquisition section (11) for acquiring a pathological image; and a classification section (12) for classifying a specimen cell as a benign cell or a malignant cell using a classification model that receives input of (i) a feature quantity of a first weighted input image which has been processed with first weighting information for emphasizing a first region of interest and (ii) a feature quantity of a second weighted input image which has been processed with second weighting information for emphasizing a second region of interest which differs from the first region of interest.

## Description

### Technical Field

The present invention relates to a classification apparatus, a training apparatus, a classification method, a training method, and a program.

### Background Art

A technique is disclosed which is related to pathological diagnosis using a trained model that carries out image recognition with respect to a pathological image upon receipt of input of the pathological image.

Patent Literature 1 discloses a configuration in which a classification result based on a feature quantity independent of a type of an organ and a detection result of a special region based on a feature quantity specific to an organ to be inspected are output based on an inspection target image obtained by imaging the organ.

### Citation List

### [Patent Literature]

[Patent Literature 1]
Japanese Patent Application Publication Tokukai No. 2012-73179

### Summary of Invention

### Technical Problem

In diagnosis for classifying, as a benign cell or a malignant cell, a specimen cell which is included as a subject in a pathological image, a technique is demanded in which a trained model that recognizes a pathological image is used to improve accuracy in classification of a specimen cell between benignancy and malignancy in pathological diagnosis.

An example aspect of the present invention is accomplished in view of the above problems, and an example object thereof is to provide a technique for improving accuracy in classification of a specimen cell between benignancy and malignancy in pathological diagnosis.

### Solution to Problem

A classification apparatus in accordance with an example aspect of the present invention includes: an acquisition means for acquiring a pathological image which includes a specimen cell as a subject; and a classification means, the classification means generating a first weighted input image by processing the pathological image with first weighting information using a first generative model that has been trained to generate, upon receipt of input of the pathological image, the first weighting information for emphasizing a first region of interest in the pathological image, the classification means generating a second weighted input image by processing the pathological image with second weighting information using a second generative model that has been trained to generate, upon receipt of input of the pathological image, the second weighting information for emphasizing a second region of interest, which differs from the first region of interest, in the pathological image, the classification means generating a feature quantity of the first weighted input image using a first feature analysis model that has been trained to generate the feature quantity of the first weighted input image upon receipt of input of the first weighted input image, the classification means generating a feature quantity of the second weighted input image using a second feature analysis model that has been trained to generate the feature quantity of the second weighted input image upon receipt of input of the second weighted input image, and the classification means classifying the specimen cell as a benign cell or a malignant cell using a classification model that has been trained to classify the specimen cell as a benign cell or a malignant cell upon receipt of input of the feature quantity of the first weighted input image and the feature quantity of the second weighted input image.

A training apparatus in accordance with an example aspect of the present invention includes: an acquisition means for acquiring (i) a pathological image which includes a specimen cell as a subject and (ii) classification information which indicates whether the specimen cell is a benign cell or a malignant cell; and a training means, the training means generating a first weighted input image by processing the pathological image with first weighting information using a first generative model that generates, upon receipt of input of the pathological image, the first weighting information for emphasizing a first region of interest in the pathological image, the training means generating a second weighted input image by processing the pathological image with second weighting information using a second generative model that generates, upon receipt of input of the pathological image, the second weighting information for emphasizing a second region of interest, which differs from the first region of interest, in the pathological image, the training means generating a feature quantity of the first weighted input image using a first feature analysis model that generates the feature quantity of the first weighted input image upon receipt of input of the first weighted input image, the training means generating a feature quantity of the second weighted input image using a second feature analysis model that generates the feature quantity of the second weighted input image upon receipt of input of the second weighted input image, the training means classifying the specimen cell as a benign cell or a malignant cell using a classification model that classifies the specimen cell as a benign cell or a malignant cell upon receipt of input of the feature quantity of the first weighted input image and the feature quantity of the second weighted input image, and the training means updating parameters of the first generative model, the second generative model, the first feature analysis model, the second feature analysis model, and the classification model based on comparison between a classification result and the classification information.

A classification method in accordance with an example aspect of the present invention includes: acquiring, by a classification apparatus, a pathological image which includes a specimen cell as a subject; generating, by the classification apparatus, a first weighted input image by processing the pathological image with first weighting information using a first generative model that has been trained to generate, upon receipt of input of the pathological image, the first weighting information for emphasizing a first region of interest in the pathological image; generating, by the classification apparatus, a second weighted input image by processing the pathological image with second weighting information using a second generative model that has been trained to generate, upon receipt of input of the pathological image, the second weighting information for emphasizing a second region of interest, which differs from the first region of interest, in the pathological image; generating, by the classification apparatus, a feature quantity of the first weighted input image using a first feature analysis model that has been trained to generate the feature quantity of the first weighted input image upon receipt of input of the first weighted input image; generating, by the classification apparatus, a feature quantity of the second weighted input image using a second feature analysis model that has been trained to generate the feature quantity of the second weighted input image upon receipt of input of the second weighted input image; and classifying, by the classification apparatus, the specimen cell as a benign cell or a malignant cell using a classification model that has been trained to classify the specimen cell as a benign cell or a malignant cell upon receipt of input of the feature quantity of the first weighted input image and the feature quantity of the second weighted input image.

A training method in accordance with an example aspect of the present invention includes: acquiring, by a training apparatus, (i) a pathological image which includes a specimen cell as a subject and (ii) classification information which indicates whether the specimen cell is a benign cell or a malignant cell; generating, by the training apparatus, a first weighted input image by processing the pathological image with first weighting information using a first generative model that generates, upon receipt of input of the pathological image, the first weighting information for emphasizing a first region of interest in the pathological image; generating, by the training apparatus, a second weighted input image by processing the pathological image with second weighting information using a second generative model that generates, upon receipt of input of the pathological image, the second weighting information for emphasizing a second region of interest, which differs from the first region of interest, in the pathological image; generating, by the training apparatus, a feature quantity of the first weighted input image using a first feature analysis model that generates the feature quantity of the first weighted input image upon receipt of input of the first weighted input image; generating, by the training apparatus, a feature quantity of the second weighted input image using a second feature analysis model that generates the feature quantity of the second weighted input image upon receipt of input of the second weighted input image; classifying, by the training apparatus, the specimen cell as a benign cell or a malignant cell using a classification model that classifies the specimen cell as a benign cell or a malignant cell upon receipt of input of the feature quantity of the first weighted input image and the feature quantity of the second weighted input image; and updating, by the training apparatus, parameters of the first generative model, the second generative model, the first feature analysis model, the second feature analysis model, and the classification model based on comparison between a classification result and the classification information.

A program in accordance with an example aspect of the present invention is a program for causing a computer to function as a classification apparatus, the program causing the computer to function as: an acquisition means for acquiring a pathological image which includes a specimen cell as a subject; and a classification means, the classification means generating a first weighted input image by processing the pathological image with first weighting information using a first generative model that has been trained to generate, upon receipt of input of the pathological image, the first weighting information for emphasizing a first region of interest in the pathological image, the classification means generating a second weighted input image by processing the pathological image with second weighting information using a second generative model that has been trained to generate, upon receipt of input of the pathological image, the second weighting information for emphasizing a second region of interest, which differs from the first region of interest, in the pathological image, the classification means generating a feature quantity of the first weighted input image using a first feature analysis model that has been trained to generate the feature quantity of the first weighted input image upon receipt of input of the first weighted input image, the classification means generating a feature quantity of the second weighted input image using a second feature analysis model that has been trained to generate the feature quantity of the second weighted input image upon receipt of input of the second weighted input image, and the classification means classifying the specimen cell as a benign cell or a malignant cell using a classification model that has been trained to classify the specimen cell as a benign cell or a malignant cell upon receipt of input of the feature quantity of the first weighted input image and the feature quantity of the second weighted input image.

A program in accordance with an example aspect of the present invention is a program for causing a computer to function as a training apparatus, the program causing the computer to function as: an acquisition means for acquiring (i) a pathological image which includes a specimen cell as a subject and (ii) classification information which indicates whether the specimen cell is a benign cell or a malignant cell; and a training means, the training means generating a first weighted input image by processing the pathological image with first weighting information using a first generative model that generates, upon receipt of input of the pathological image, the first weighting information for emphasizing a first region of interest in the pathological image, the training means generating a second weighted input image by processing the pathological image with second weighting information using a second generative model that generates, upon receipt of input of the pathological image, the second weighting information for emphasizing a second region of interest, which differs from the first region of interest, in the pathological image, the training means generating a feature quantity of the first weighted input image using a first feature analysis model that generates the feature quantity of the first weighted input image upon receipt of input of the first weighted input image, the training means generating a feature quantity of the second weighted input image using a second feature analysis model that generates the feature quantity of the second weighted input image upon receipt of input of the second weighted input image, the training means classifying the specimen cell as a benign cell or a malignant cell using a classification model that classifies the specimen cell as a benign cell or a malignant cell upon receipt of input of the feature quantity of the first weighted input image and the feature quantity of the second weighted input image, and the training means updating parameters of the first generative model, the second generative model, the first feature analysis model, the second feature analysis model, and the classification model based on comparison between a classification result and the classification information.

### Advantageous Effects of Invention

According to an example aspect of the present invention, it is possible to improve accuracy in classification of a specimen cell between benignancy and malignancy in pathological diagnosis.

### Brief Description of Drawings

Fig. 1 is a block diagram illustrating a configuration of a classification apparatus in accordance with a first example embodiment of the present invention.
Fig. 2 is a flowchart illustrating a flow of a classification method in accordance with the first example embodiment of the present invention.
Fig. 3 is a block diagram illustrating a configuration of a training apparatus in accordance with the first example embodiment of the present invention.
Fig. 4 is a flowchart illustrating a flow of a training method in accordance with the first example embodiment of the present invention.
Fig. 5 is a block diagram illustrating a configuration of a classification apparatus in accordance with a second example embodiment of the present invention.
Fig. 6 is a schematic diagram illustrating a flow of a process which is carried out by a classification section in accordance with the second example embodiment of the present invention.
Fig. 7 is a block diagram illustrating an example hardware configuration of the classification apparatus and the training apparatus in accordance with the example embodiments of the present invention.

### Example Embodiments

### [First example embodiment]

The following description will discuss a first example embodiment of the present invention in detail, with reference to the drawings. The present example embodiment is a basic form of example embodiments described later.

### (Overview of classification apparatus 1)

A classification apparatus 1 in accordance with the present example embodiment is an apparatus which classifies a specimen cell as a benign cell or a malignant cell.

For example, the classification apparatus 1 first generates an image (hereinafter, referred to as "input image obtained by processing a pathological image with weighting information") which has been obtained by carrying out a weighting process with respect to a pathological image using weighting information, by use of a generative model that has been trained to generate, upon receipt of input of a pathological image which includes a specimen cell as a subject, weighting information for emphasizing a region of interest in the pathological image.

Next, the classification apparatus 1 generates a feature quantity of the weighted input image by using a feature analysis model that has been trained to generate, upon receipt of input of a weighted input image, a feature quantity of the weighted input image.

Then, the classification apparatus 1 classifies the specimen cell as a benign cell or a malignant cell by using a classification model that has been trained to classify a specimen cell as a benign cell or a malignant cell upon receipt of input of a feature quantity of a weighted input image. The classification apparatus 1 can be used, for example, in cytodiagnosis in rapid on-site evaluation (ROSE).

Here, examples of the weighting information include an attention map that indicates a region of interest by setting a weight for each region. The following description will discuss a case in which the weighting information is an attention map.

The number of generative models and feature analysis models is not particularly limited. The following description will discuss a case in which, as an example, two generative models (hereinafter referred to as a first generative model and a second generative model) and two feature analysis models (a first feature analysis model and a second feature analysis model) are used.

In the following description, attention maps generated by the first generative model and the second generative model are referred to as a first attention map and a second attention map, respectively. Moreover, input images processed with the first attention map and the second attention map are referred to as a first weighted input image and a second weighted input image, respectively. Moreover, regions of interest, that is, regions emphasized in the attention maps in the first weighted input image and the second weighted input image are referred to as a first region of interest and a second region of interest, respectively.

Specific configurations of the generative model, the feature analysis model, and the classification model do not limit the present example embodiment. For example, it is possible to use a deep neural network (such as a convolution neural network (CNN)) having a convolution layer. Examples of the convolution layer include, but not limited to, layers having the following configurations.
- Batch normalization layer
- Activation layer (e.g., a rectified linear unit (ReLU) layer)
- Two-dimensional convolution layer
- Pooling layer

### (Configuration of classification apparatus 1)

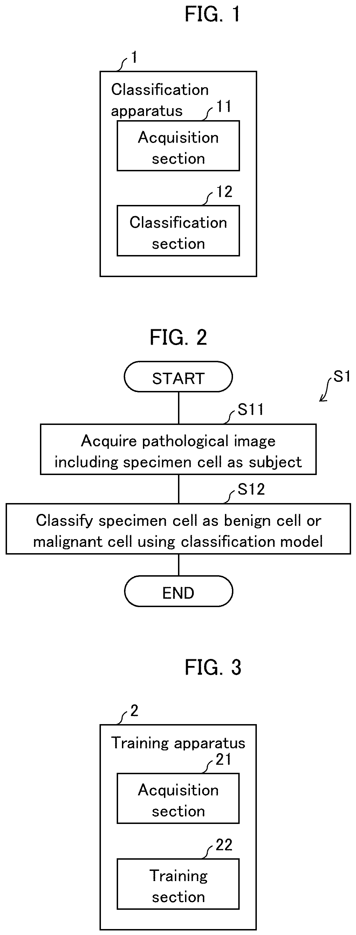

The following description will discuss a configuration of the classification apparatus 1 in accordance with the present example embodiment, with reference to Fig. 1. Fig. 1 is a block diagram illustrating a configuration of the classification apparatus 1 in accordance with the present example embodiment.

As illustrated in Fig. 1, the classification apparatus 1 includes an acquisition section 11 and a classification section 12. In the present example embodiment, the acquisition section 11 and the classification section 12 are components for implementing the acquisition means and the classification means, respectively.

The acquisition section 11 acquires a pathological image which includes a specimen cell as a subject. The acquisition section 11 supplies the acquired pathological image to the classification section 12.

The classification section 12 generates a first weighted input image by processing the pathological image with the first attention map using the first generative model.

Moreover, the classification section 12 generates a second weighted input image by processing the pathological image with the second attention map using the second generative model.

Moreover, the classification section 12 generates a feature quantity of the first weighted input image using the first feature analysis model.

Moreover, the classification section 12 generates a feature quantity of the second weighted input image using the second feature analysis model.

Moreover, the classification section 12 classifies the specimen cell as a benign cell or a malignant cell using the classification model.

As described above, the classification apparatus 1 in accordance with the present example embodiment employs the configuration of including: the acquisition section 11 for acquiring a pathological image which includes a specimen cell as a subject; and the classification section 12, the classification section 12 generating a first weighted input image by processing the pathological image with a first attention map using a first generative model that has been trained to generate, upon receipt of input of the pathological image, the first attention map for emphasizing a first region of interest in the pathological image, the classification section 12 generating a second weighted input image by processing the pathological image with a second attention map using a second generative model that has been trained to generate, upon receipt of input of the pathological image, the second attention map for emphasizing a second region of interest, which differs from the first region of interest, in the pathological image, the classification section 12 generating a feature quantity of the first weighted input image using a first feature analysis model that has been trained to generate the feature quantity of the first weighted input image upon receipt of input of the first weighted input image, the classification section 12 generating a feature quantity of the second weighted input image using a second feature analysis model that has been trained to generate the feature quantity of the second weighted input image upon receipt of input of the second weighted input image, and the classification section 12 classifying the specimen cell as a benign cell or a malignant cell using a classification model that has been trained to classify the specimen cell as a benign cell or a malignant cell upon receipt of input of the feature quantity of the first weighted input image and the feature quantity of the second weighted input image.

Therefore, the classification apparatus 1 in accordance with the present example embodiment classifies a specimen cell as a benign cell or a malignant cell, while paying attention to each of the first region of interest and the second region of interest which are different from each other in the pathological image. For example, the classification apparatus 1 in accordance with the present example embodiment can vary a region of interest depending on a type of cell included in a pathological image in pathological diagnosis for classifying a specimen cell as a benign cell or a malignant cell. Therefore, according to the classification apparatus 1 in accordance with the present example embodiment, it is possible to bring about an example advantage of improving accuracy in classification of a specimen cell between benignancy and malignancy in pathological diagnosis.

### (Flow of classification method S1)

The following description will discuss a flow of a classification method S1 in accordance with the present example embodiment, with reference to Fig. 2. Fig. 2 is a flowchart illustrating the flow of the classification method S1 in accordance with the present example embodiment.

### (Step S11)

In step S11, the acquisition section 11 acquires a pathological image which includes a specimen cell as a subject. The acquisition section 11 supplies the acquired pathological image to the classification section 12.

### (Step S12)

In step S12, the classification section 12 generates a first weighted input image by processing the pathological image with the first attention map using the first generative model.

Moreover, the classification section 12 generates a second weighted input image by processing the pathological image with the second attention map using the second generative model.

Moreover, the classification section 12 generates a feature quantity of the first weighted input image using the first feature analysis model.

Moreover, the classification section 12 generates a feature quantity of the second weighted input image using the second feature analysis model.

Moreover, the classification section 12 classifies the specimen cell as a benign cell or a malignant cell using the classification model.

As described above, the classification method S1 in accordance with the present example embodiment employs the configuration of including: acquiring a pathological image which includes a specimen cell as a subject; generating a first weighted input image by processing the pathological image with a first attention map using a first generative model that has been trained to generate, upon receipt of input of the pathological image, the first attention map for emphasizing a first region of interest in the pathological image; generating a second weighted input image by processing the pathological image with a second attention map using a second generative model that has been trained to generate, upon receipt of input of the pathological image, the second attention map for emphasizing a second region of interest, which differs from the first region of interest, in the pathological image; generating a feature quantity of the first weighted input image using a first feature analysis model that has been trained to generate the feature quantity of the first weighted input image upon receipt of input of the first weighted input image; generating a feature quantity of the second weighted input image using a second feature analysis model that has been trained to generate the feature quantity of the second weighted input image upon receipt of input of the second weighted input image; and classifying the specimen cell as a benign cell or a malignant cell using a classification model that has been trained to classify the specimen cell as a benign cell or a malignant cell upon receipt of input of the feature quantity of the first weighted input image and the feature quantity of the second weighted input image. Therefore, according to the classification method S1 in accordance with the present example embodiment, an example advantage similar to that of the foregoing classification apparatus 1 is brought about.

### (Overview of training apparatus 2)

A training apparatus 2 in accordance with the present example embodiment is an apparatus which updates parameters of a first generative model, a second generative model, a first feature analysis model, a second feature analysis model, and a classification model.

For example, the training apparatus 2 acquires (i) a pathological image which includes a specimen cell as a subject and (ii) classification information (correct answer label) which indicates whether the specimen cell is a benign cell or a malignant cell, and updates parameters of each of the models based on comparison between a result of classification by the classification model and the classification information.

The first generative model, the second generative model, the first feature analysis model, the second feature analysis model, and the classification model are as described above.

### (Configuration of training apparatus 2)

The following description will discuss a configuration of the training apparatus 2 in accordance with the present example embodiment, with reference to Fig. 3. Fig. 3 is a block diagram illustrating the configuration of the training apparatus 2 in accordance with the present example embodiment.

As illustrated in Fig. 3, the training apparatus 2 includes an acquisition section 21 and a training section 22. In the present example embodiment, the acquisition section 21 and the training section 22 are components for implementing the acquisition means and the training means, respectively.

The acquisition section 21 acquires (i) a pathological image which includes a specimen cell as a subject and (ii) classification information which indicates whether the specimen cell is a benign cell or a malignant cell. The acquisition section 11 supplies the acquired pathological image and classification information to the training section 22.

The training section 22 updates parameters of the first generative model, the second generative model, the first feature analysis model, the second feature analysis model, and the classification model based on comparison between a result of classification by the classification model and the classification information, and thus trains the first generative model, the second generative model, the first feature analysis model, the second feature analysis model, and the classification model. The training apparatus 2 can be used, for example, to train a classification model used in cytodiagnosis in rapid on-site evaluation (ROSE).

As described above, the training apparatus 2 in accordance with the present example embodiment employs the configuration of including: the acquisition section 21 for acquiring (i) a pathological image which includes a specimen cell as a subject and (ii) classification information which indicates whether the specimen cell is a benign cell or a malignant cell; and the training section 22, the training section 22 generating a first weighted input image by processing the pathological image with first weighting information using a first generative model that generates, upon receipt of input of the pathological image, the first weighting information for emphasizing a first region of interest in the pathological image, the training section 22 generating a second weighted input image by processing the pathological image with second weighting information using a second generative model that generates, upon receipt of input of the pathological image, the second weighting information for emphasizing a second region of interest, which differs from the first region of interest, in the pathological image, the training section 22 generating a feature quantity of the first weighted input image using a first feature analysis model that generates the feature quantity of the first weighted input image upon receipt of input of the first weighted input image, the training section 22 generating a feature quantity of the second weighted input image using a second feature analysis model that generates the feature quantity of the second weighted input image upon receipt of input of the second weighted input image, the training section 22 classifying the specimen cell as a benign cell or a malignant cell using a classification model that classifies the specimen cell as a benign cell or a malignant cell upon receipt of input of the feature quantity of the first weighted input image and the feature quantity of the second weighted input image, and the training section 22 updating parameters of the first generative model, the second generative model, the first feature analysis model, the second feature analysis model, and the classification model based on comparison between a classification result and the classification information.

Therefore, the training apparatus 2 in accordance with the present example embodiment can accurately train the first generative model that generates a first attention map, the second generative model that generates a second attention map, the first feature analysis model that generates a feature quantity of a first weighted input image, the second feature analysis model that generates a feature quantity of the second weighted input image, and the classification model that classifies a specimen cell as a benign cell or a malignant cell. Therefore, according to the training apparatus 2 in accordance with the present example embodiment, it is possible to bring about an example advantage of improving accuracy in classification of a specimen cell between benignancy and malignancy in pathological diagnosis.

### (Flow of training method S2)

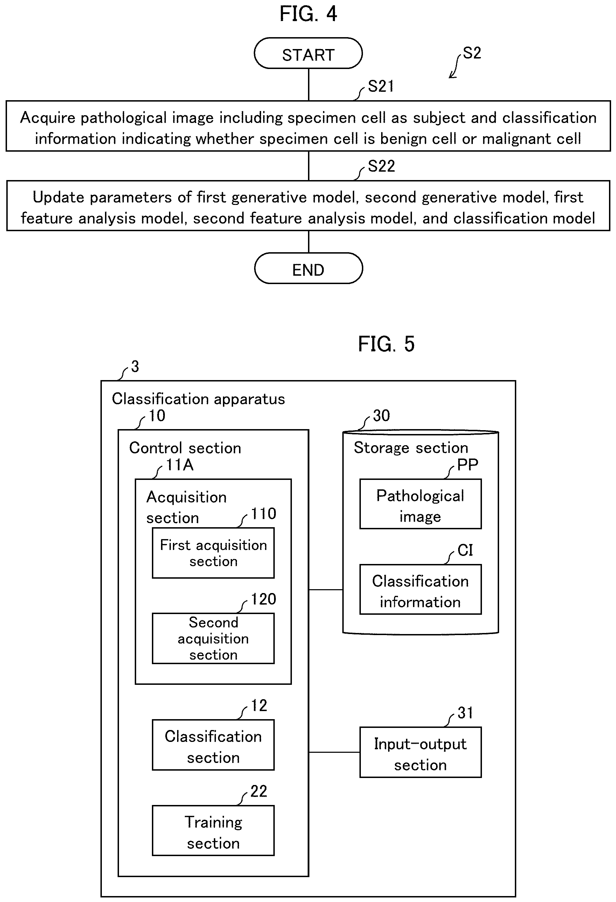

The following description will discuss a flow of a training method S2 in accordance with the present example embodiment, with reference to Fig. 4. Fig. 2 is a flowchart illustrating the flow of the training method S2 in accordance with the present example embodiment.

### (Step S21)

In step S21, the acquisition section 21 acquires (i) a pathological image which includes a specimen cell as a subject and (ii) classification information (correct answer label) which indicates whether the specimen cell is a benign cell or a malignant cell. The acquisition section 11 supplies the acquired pathological image and classification information to the training section 22.

### (Step S22)

The training section 22 updates parameters of the first generative model, the second generative model, the first feature analysis model, the second feature analysis model, and the classification model based on comparison between a result of classification by the first generative model and the classification model and classification information.

As described above, the training method S2 in accordance with the present example embodiment employs the configuration of including: acquiring (i) a pathological image which includes a specimen cell as a subject and (ii) classification information which indicates whether the specimen cell is a benign cell or a malignant cell; generating a first weighted input image by processing the pathological image with first weighting information using a first generative model that generates, upon receipt of input of the pathological image, the first weighting information for emphasizing a first region of interest in the pathological image; generating a second weighted input image by processing the pathological image with second weighting information using a second generative model that generates, upon receipt of input of the pathological image, the second weighting information for emphasizing a second region of interest, which differs from the first region of interest, in the pathological image; generating a feature quantity of the first weighted input image using a first feature analysis model that generates the feature quantity of the first weighted input image upon receipt of input of the first weighted input image; generating a feature quantity of the second weighted input image using a second feature analysis model that generates the feature quantity of the second weighted input image upon receipt of input of the second weighted input image; classifying the specimen cell as a benign cell or a malignant cell using a classification model that classifies the specimen cell as a benign cell or a malignant cell upon receipt of input of the feature quantity of the first weighted input image and the feature quantity of the second weighted input image; and updating parameters of the first generative model, the second generative model, the first feature analysis model, the second feature analysis model, and the classification model based on comparison between a classification result and the classification information. Therefore, according to the training method S2 in accordance with the present example embodiment, an example advantage similar to that of the foregoing training apparatus 2 is brought about.

### [Second example embodiment]

The following description will discuss a second example embodiment of the present invention in detail, with reference to the drawings. The same reference numerals are given to constituent elements which have functions identical with those described in the first example embodiment, and descriptions as to such constituent elements are omitted as appropriate.

### (Overview of classification apparatus 1A)

A classification apparatus 1A in accordance with the present example embodiment is an apparatus which classifies a specimen cell as a benign cell or a malignant cell.

For example, the classification apparatus 1A first generates an image (hereinafter, referred to as "input image obtained by processing a pathological image with weighting information") which has been obtained by carrying out a weighting process with respect to a pathological image using weighting information, by use of a generative model that has been trained to generate, upon receipt of input of a pathological image which includes a specimen cell as a subject, weighting information for emphasizing a region of interest in the pathological image.

Next, the classification apparatus 1A generates a feature quantity of the weighted input image by using a feature analysis model that has been trained to generate, upon receipt of input of a weighted input image, a feature quantity of the weighted input image.

Then, the classification apparatus 1A classifies the specimen cell as a benign cell or a malignant cell by using a classification model that has been trained to classify a specimen cell as a benign cell or a malignant cell upon receipt of input of a feature quantity of a weighted input image.

A specimen cell which is included as a subject in a pathological image is a cell which has been stained in advance by a staining method (such as Diff-Quik staining or Papanicolaou staining) that stains at least cytoplasm.

In the present example embodiment also, the following description will discuss a case in which the weighting information is an attention map.

In the present example embodiment also, the number of generative models and feature analysis models is not particularly limited. The following description will discuss a case in which, as an example, two generative models (hereinafter referred to as a first generative model and a second generative model) and two feature analysis models (a first feature analysis model and a second feature analysis model) are used.

The first attention map, the second attention map, the first weighted input image, the second weighted input image, the first region of interest, the second region of interest, the generative model, the feature analysis model, and the classification model are as in the foregoing example embodiment.

Here, the first region of interest in the first weighted input image and the second region of interest in the second weighted input image are not particularly limited as long as the regions are different from each other. For example, at least cytoplasm of a specimen cell is stained, and the first region of interest and the second region of interest are different in staining intensity from each other.

As an example of the first region of interest and the second region of interest, at least cytoplasm of a specimen cell is stained, and in the weighted input image, a region having a staining intensity equal to or more than a first threshold value may be set as the first region of interest, and a region having a staining intensity that is equal to or more than a second threshold value, which is lower than the first threshold value, and that is lower than the first threshold value may be set as the second region of interest.

As another example of the first region of interest and the second region of interest, the first region of interest is a region having a staining intensity higher than that of the second region of interest, and the second region of interest is a region including stained cilia.

As yet another example of the first region of interest and the second region of interest, the first region of interest is a region (in other words, a region close to the center of a cell or the center of a nucleolus) in which a distance from a pixel having the highest staining intensity in a cell is shorter than a first predetermined length, and the second region of interest is a region in which a distance from the center of the cell or the center of the nucleolus is shorter than a second predetermined length, which is longer than the first predetermined length, and is longer than the first predetermined length.

The classification apparatus 1A is an apparatus which updates parameters of the first generative model, the second generative model, the first feature analysis model, the second feature analysis model, and the classification model. In other words, the classification apparatus 1A includes also the configuration of the training apparatus 2 described above.

For example, the classification apparatus 1A acquires (i) a pathological image which includes a specimen cell as a subject and (ii) classification information which indicates whether the specimen cell is a benign cell or a malignant cell, and updates parameters of each of the models based on comparison between a result of classification by the classification model and the classification information.

### (Configuration of classification apparatus 1A)

As illustrated in Fig. 5, the classification apparatus 1A in accordance with the present example embodiment includes a control section 10, a storage section 30, and an input-output section 31.

In the storage section 30, data referred to by the control section 10 (described later) is stored. Examples of the data stored in the storage section 30 include a pathological image PP and classification information CI.

The input-output section 31 is an interface via which data is acquired from another apparatus connected to the classification apparatus 1A and data is outputted to another apparatus which is connected to the classification apparatus 1A. The input-output section 31 supplies data acquired from another apparatus to the control section 10 and outputs data supplied from the control section 10 to another apparatus.

The input-output section 31 may be a communication module that communicates with other apparatuses via a network. A specific configuration of the network does not limit the present example embodiment, and can be, for example, a wireless local area network (LAN), a wired LAN, a wide area network (WAN), a public network, a mobile data communication network, or a combination of these networks.

### (Control section 10)

The control section 10 controls constituent elements included in the classification apparatus 1A. As illustrated in Fig. 5, the control section 10 functions also as an acquisition section 11A, a classification section 12, and a training section 22. In the present example embodiment, the acquisition section 11A, the classification section 12, and the training section 22 are components for implementing the acquisition means, the classification means, and the training means, respectively.

The acquisition section 11A acquires data supplied from the input-output section 31. The acquisition section 11A causes the storage section 30 to store the acquired data. As illustrated in Fig. 5, the acquisition section 11A includes a first acquisition section 110 and a second acquisition section 120.

The first acquisition section 110 acquires a pathological image PP which includes a specimen cell as a subject. In other words, the first acquisition section 110 includes the function of the acquisition section 11 in the foregoing example embodiment.

The second acquisition section 120 acquires (i) a pathological image which includes a specimen cell as a subject and (ii) classification information which indicates whether the specimen cell is a benign cell or a malignant cell. In other words, the second acquisition section 120 includes the function of the acquisition section 21 in the foregoing example embodiment.

The classification section 12 generates a first weighted input image by processing the pathological image with the first attention map using the first generative model.

Moreover, the classification section 12 generates a second weighted input image by processing the pathological image with the second attention map using the second generative model.

Moreover, the classification section 12 generates a feature quantity of the first weighted input image using the first feature analysis model.

Moreover, the classification section 12 generates a feature quantity of the second weighted input image using the second feature analysis model.

Moreover, the classification section 12 classifies the specimen cell as a benign cell or a malignant cell using the classification model.

The training section 22 updates parameters of the first generative model, the second generative model, the first feature analysis model, the second feature analysis model, and the classification model based on comparison between a result of classification by the classification model and classification information.

### (Overview of process carried out by classification section 12)

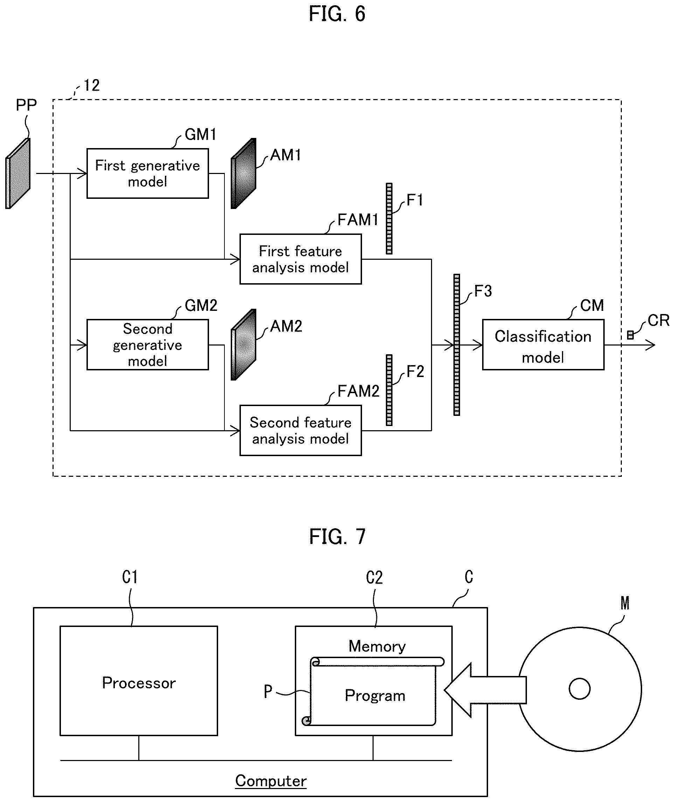

The following description will discuss a process which is carried out by the classification section 12, with reference to Fig. 6. Fig. 6 is a schematic diagram illustrating a flow of a process which is carried out by the classification section 12 in accordance with the present example embodiment.

First, the classification section 12 acquires, from the storage section 30, a pathological image PP which includes a specimen cell as a subject. The classification section 12 supplies the acquired pathological image PP to a first generative model GM1 and a second generative model GM2.

Next, the classification section 12 generates a weighted input image which has been processed with an attention map AM generated by the generative model GM.

For example, in a case of an attention map in which a region of interest is represented using a weight of 0 to 1, the classification section 12 multiplies pixel values corresponding in a first attention map AM1, which has been generated by the first generative model GM1 upon receipt of input of the pathological image PP, and in the pathological image PP. Thus, the classification section 12 generates a first weighted input image which has been processed with the first attention map AM1.

Similarly, the classification section 12 multiplies pixel values corresponding in a second attention map AM2, which has been generated by the second generative model GM2 upon receipt of input of the pathological image PP, and in the pathological image PP. Thus, the classification section 12 generates a second weighted input image which has been processed with the second attention map AM2.

Subsequently, the classification section 12 generates a feature quantity F3 by combining (i) a feature quantity F1 of the first weighted input image generated by the first feature analysis model upon receipt of input of the generated first weighted input image and (ii) a feature quantity F2 of the second weighted input image generated by the second feature analysis model upon receipt of input of the generated second weighted input image. For example, the feature quantities F1 and F2 are typically 1024 vectors.

Then, the classification section 12 inputs the feature quantity F3 into a classification model CM and classifies the specimen cell as a benign cell or a malignant cell with reference to a classification result CR by the classification model.

For example, in a case where the classification result CR indicates that a specimen cell which is included as a subject in the pathological image PP is a benign cell, the classification section 12 classifies, as a benign cell, the specimen cell which is included as a subject in the pathological image PP. Meanwhile, in a case where the classification result CR indicates that a specimen cell which is included as a subject in the pathological image PP is a malignant cell, the classification section 12 classifies, as a malignant cell, the specimen cell which is included as a subject in the pathological image PP.

Here, the classification section 12 may skip the processes by the first generative model GM1 and the second generative model GM2 to generate attention maps.

For example, in a case of an attention map in which a region of interest is represented using a weight of 0 to 1, attention maps respectively generated by the first generative model GM1 and the second generative model GM2 may be set so that the weight is 1 in all regions of the pathological image PP.

The classification section 12 generates a weighted input image by multiplying pixel values corresponding in the attention map AM and in the pathological image PP. Therefore, in a case where the attention map is set so that the weight is 1 in all regions of the pathological image PP, the classification section 12 skips the processes by the first generative model GM1 and the second generative model GM2 and inputs the pathological image PP as a weighted input image into the feature analysis model.

### (Process carried out by training section 22)

The following description will discuss a process which is carried out by the training section 22.

For example, in order to accurately determine whether a specimen cell is a malignant cell or a benign cell, the training section 22 carries out training so that a first attention map AM1 for emphasizing a first region of interest with a high staining intensity and a second attention map AM2 for emphasizing a second region of interest having a staining intensity which is lower than that of the first region of interest and is moderate are generated.

As another example, in order to accurately determine whether a specimen cell is a malignant cell or a benign cell, the training section 22 carries out training so that a second attention map AM2 for emphasizing a second region including stained cilia and a first attention map AM1 for emphasizing a first region having a staining intensity which is higher than that of the second region are generated.

The following description will discuss, for example, a configuration in a case where a first attention map AM1 is generated in which a region having a staining intensity equal to or more than a first threshold value is set to be a first region of interest and the second generative model GM2 generates a second attention map AM2 in which a region having a staining intensity which is equal to or more than a second threshold value, which is lower than the first threshold value, and is lower than the first threshold value is set to be a second region of interest.

First, the training section 22 sets initial values for the first generative model GM1 and the second generative model GM2. For example, the training section 22 sets, for the first generative model GM1, an initial value with which a weight of a region (e.g., a region having a pixel value of 180 or more in an image with 256 levels of gray) that is a first region of interest and has a staining intensity equal to or more than the first threshold value is set to 1, and weights in the other regions are set to 0.

Moreover, the training section 22 sets, for the second generative model GM2, an initial value with which a weight of a region (e.g., a region having a pixel value of 64 to 179 in an image with 256 levels of gray) that is a second region of interest and has a staining intensity which is equal to or more than the second threshold value lower than the first threshold value and is lower than the first threshold value is set to 1, and weights in the other regions are set to 0. Thus, the training section 22 may set the initial values of the first region of interest and the second region of interest to be relatively higher than the other regions in the first attention map AM1 and the second attention map AM2.

Here, the training section 22 may further divide a region other than the first region of interest and a region other than the second region of interest described above and set weights. For example, the training section 22 may set, for the second generative model GM2, an initial value with which a weight of a region (e.g., a region having a pixel value of 180 or more in an image with 256 levels of gray) having a color intensity equal to or more than the first threshold value is set to 0.5.

The training section 22 may vary an initial value of a weight in accordance with pixel values in the first region of interest and the second region of interest. In other words, the training section 22 may set initial values of the first attention map AM1 and the second attention map AM2 so that the first region of interest and the second region of interest are different in staining intensity from each other. For example, the training section 22 may set the initial value for the first generative model GM1 as follows: an initial value of a region having a pixel value of 180 to 209 is set to 1, an initial value of a region having a pixel value of 210 to 229 is set to 0.9, and an initial value of a region having a pixel value of 230 to 255 is set to 0.8. The training section 22 may vary an initial value of a weight in accordance with pixel values similarly in a region other than the first region of interest and a region other than the second region of interest.

Next, the training section 22 updates parameters of the first generative model, the second generative model, the first feature analysis model, the second feature analysis model, and the classification model based on comparison between a classification result CR and classification information CI using an optimization algorithm such as a steepest descent method.

For example, in a case where the classification result CR indicates that a specimen cell is a benign cell and the classification information CI indicates that the specimen cell is a malignant cell, the training section 22 updates parameters of the first generative model, the second generative model, the first feature analysis model, the second feature analysis model, and the classification model so that, in a case where the pathological image PP is input into the classification section 12, the classification result CR outputs a classification result indicating that the specimen cell is a malignant cell.

By repeating the above process, it is possible to train the first generative model, the second generative model, the first feature analysis model, the second feature analysis model, and the classification model with high accuracy.

### (Example advantage 1 of classification apparatus 1A)

As described above, the classification apparatus 1A in accordance with the present example embodiment employs the configuration of including: the acquisition section 11A for acquiring a pathological image PP which includes a specimen cell as a subject; and the classification section 12 for classifying the specimen cell as a benign cell or a malignant cell based on a feature quantity F1 of a first weighted input image generated using a first attention map AM1 for emphasizing a first region of interest and a feature quantity F2 of a second weighted input image generated using a second attention map AM2 for emphasizing a second region of interest.

For example, in a case where a certain cell can be classified as a benign cell or a malignant cell by focusing on a region having a high staining intensity and the other cell different from the certain cell can be classified as a benign cell or a malignant cell by focusing on a region having a relatively low staining intensity, the classification apparatus 1A in accordance with the present example embodiment can set the first region of interest and the second region of interest to be regions having different staining intensities from each other.

Therefore, according to the classification apparatus 1A in accordance with the present example embodiment, it is possible to improve accuracy in classification of a specimen cell between benignancy and malignancy in pathological diagnosis.

As another example, in a case where a certain cell can be classified as a malignant cell by focusing on the vicinity of a nucleolus and the certain cell can be classified as a benign cell by focusing on cilia in the other cell different from the certain cell, the classification apparatus 1A in accordance with the present example embodiment can set the first region of interest to be a region having a staining intensity higher than that of the second region of interest and set the second region of interest to be a region including stained cilia.

Therefore, according to the classification apparatus 1A in accordance with the present example embodiment, it is possible to improve accuracy in classification of a specimen cell between benignancy and malignancy in pathological diagnosis.

### (Example advantage 2 of classification apparatus 1A)

The classification apparatus 1A in accordance with the present example embodiment updates parameters of the first generative model, the second generative model, the first feature analysis model, the second feature analysis model, and the classification model based on comparison between a classification result CR and classification information CI. Therefore, according to the training apparatus 2 in accordance with the present example embodiment, it is possible to accurately train the first generative model, the second generative model, the first feature analysis model, the second feature analysis model, and the classification model. This makes it possible to bring about an example advantage of improving accuracy in classification of a specimen cell between benignancy and malignancy in pathological diagnosis.

### [Software implementation example]

Some or all of the functions of each of the classification apparatuses 1 and 3 and the training apparatus 2 may be implemented by hardware such as an integrated circuit (IC chip), or may be implemented by software.

In the latter case, the classification apparatuses 1 and 3 and the training apparatus 2 are implemented by, for example, a computer that executes instructions of a program that is software implementing the foregoing functions. Fig. 7 illustrates an example of such a computer (hereinafter, referred to as "computer C"). The computer C includes at least one processor C1 and at least one memory C2. The memory C2 stores a program P for causing the computer C to operate as the classification apparatuses 1 and 3 and the training apparatus 2. The processor C1 of the computer C retrieves the program P from the memory C2 and executes the program P, so that the functions of the classification apparatuses 1 and 3 and the training apparatus 2 are implemented.

As the processor C1, for example, it is possible to use a central processing unit (CPU), a graphic processing unit (GPU), a digital signal processor (DSP), a micro processing unit (MPU), a floating point number processing unit (FPU), a physics processing unit (PPU), a microcontroller, or a combination of these. Examples of the memory C2 include a flash memory, a hard disk drive (HDD), a solid state drive (SSD), and a combination thereof.

Note that the computer C may further include a random access memory (RAM) in which the program P is loaded in a case where the program P is executed and in which various kinds of data are temporarily stored. The computer C may further include a communication interface for carrying out transmission and reception of data with other apparatuses. The computer C may further include an input-output interface for connecting input-output apparatuses such as a keyboard, a mouse, a display and a printer.

The program P can be stored in a computer C-readable, non-transitory, and tangible storage medium M. The storage medium M can be, for example, a tape, a disk, a card, a semiconductor memory, a programmable logic circuit, or the like. The computer C can obtain the program P via the storage medium M. The program P can be transmitted via a transmission medium. The transmission medium can be, for example, a communication network, a broadcast wave, or the like. The computer C can obtain the program P also via such a transmission medium.

### [Additional remark 1]

The present invention is not limited to the foregoing example embodiments, but may be altered in various ways by a skilled person within the scope of the claims. For example, the present invention also encompasses, in its technical scope, any example embodiment derived by appropriately combining technical means disclosed in the foregoing example embodiments.

### [Additional remark 2]

Some or all of the foregoing example embodiments can also be described as below. Note, however, that the present invention is not limited to the following supplementary notes.

### (Supplementary note 1)

A classification apparatus, including: an acquisition means for acquiring a pathological image which includes a specimen cell as a subject; and a classification means, the classification means generating a first weighted input image by processing the pathological image with first weighting information using a first generative model that has been trained to generate, upon receipt of input of the pathological image, the first weighting information for emphasizing a first region of interest in the pathological image, the classification means generating a second weighted input image by processing the pathological image with second weighting information using a second generative model that has been trained to generate, upon receipt of input of the pathological image, the second weighting information for emphasizing a second region of interest, which differs from the first region of interest, in the pathological image, the classification means generating a feature quantity of the first weighted input image using a first feature analysis model that has been trained to generate the feature quantity of the first weighted input image upon receipt of input of the first weighted input image, the classification means generating a feature quantity of the second weighted input image using a second feature analysis model that has been trained to generate the feature quantity of the second weighted input image upon receipt of input of the second weighted input image, and the classification means classifying the specimen cell as a benign cell or a malignant cell using a classification model that has been trained to classify the specimen cell as a benign cell or a malignant cell upon receipt of input of the feature quantity of the first weighted input image and the feature quantity of the second weighted input image.

### (Supplementary note 2)

The classification apparatus according to supplementary note 1, in which: at least cytoplasm of the specimen cell is stained; and the first region of interest and the second region of interest are different in staining intensity from each other.

### (Supplementary note 3)

The classification apparatus according to supplementary note 2, in which: the second region of interest is a region including stained cilia, and the first region of interest is a region having a staining intensity higher than that of the second region of interest.

### (Supplementary note 4)

A training apparatus, including: an acquisition means for acquiring (i) a pathological image which includes a specimen cell as a subject and (ii) classification information which indicates whether the specimen cell is a benign cell or a malignant cell; and a training means, the training means generating a first weighted input image by processing the pathological image with first weighting information using a first generative model that generates, upon receipt of input of the pathological image, the first weighting information for emphasizing a first region of interest in the pathological image, the training means generating a second weighted input image by processing the pathological image with second weighting information using a second generative model that generates, upon receipt of input of the pathological image, the second weighting information for emphasizing a second region of interest, which differs from the first region of interest, in the pathological image, the training means generating a feature quantity of the first weighted input image using a first feature analysis model that generates the feature quantity of the first weighted input image upon receipt of input of the first weighted input image, the training means generating a feature quantity of the second weighted input image using a second feature analysis model that generates the feature quantity of the second weighted input image upon receipt of input of the second weighted input image, the training means classifying the specimen cell as a benign cell or a malignant cell using a classification model that classifies the specimen cell as a benign cell or a malignant cell upon receipt of input of the feature quantity of the first weighted input image and the feature quantity of the second weighted input image, and the training means updating parameters of the first generative model, the second generative model, the first feature analysis model, the second feature analysis model, and the classification model based on comparison between a classification result and the classification information.

### (Supplementary note 5)

The training apparatus according to supplementary note 4, in which: at least cytoplasm of the specimen cell is stained; and the training means sets initial values of the first weighting information and the second weighting information so that the first region of interest and the second region of interest are different in staining intensity from each other.

### (Supplementary note 6)

The training apparatus according to supplementary note 5, in which: the second region of interest is a region including stained cilia, and the first region of interest is a region having a staining intensity higher than that of the second region of interest; and the training means sets the initial values of the first region of interest and the second region of interest in the first weighting information and the second weighting information to be relatively higher than those of the other regions.

### (Supplementary note 7)

A classification method, including: acquiring, by a classification apparatus, a pathological image which includes a specimen cell as a subject; generating, by the classification apparatus, a first weighted input image by processing the pathological image with first weighting information using a first generative model that has been trained to generate, upon receipt of input of the pathological image, the first weighting information for emphasizing a first region of interest in the pathological image; generating, by the classification apparatus, a second weighted input image by processing the pathological image with second weighting information using a second generative model that has been trained to generate, upon receipt of input of the pathological image, the second weighting information for emphasizing a second region of interest, which differs from the first region of interest, in the pathological image; generating, by the classification apparatus, a feature quantity of the first weighted input image using a first feature analysis model that has been trained to generate the feature quantity of the first weighted input image upon receipt of input of the first weighted input image; generating, by the classification apparatus, a feature quantity of the second weighted input image using a second feature analysis model that has been trained to generate the feature quantity of the second weighted input image upon receipt of input of the second weighted input image; and classifying, by the classification apparatus, the specimen cell as a benign cell or a malignant cell using a classification model that has been trained to classify the specimen cell as a benign cell or a malignant cell upon receipt of input of the feature quantity of the first weighted input image and the feature quantity of the second weighted input image.

### (Supplementary note 8)

A training method, including: acquiring, by a training apparatus, (i) a pathological image which includes a specimen cell as a subject and (ii) classification information which indicates whether the specimen cell is a benign cell or a malignant cell; generating, by the training apparatus, a first weighted input image by processing the pathological image with first weighting information using a first generative model that generates, upon receipt of input of the pathological image, the first weighting information for emphasizing a first region of interest in the pathological image; generating, by the training apparatus, a second weighted input image by processing the pathological image with second weighting information using a second generative model that generates, upon receipt of input of the pathological image, the second weighting information for emphasizing a second region of interest, which differs from the first region of interest, in the pathological image; generating, by the training apparatus, a feature quantity of the first weighted input image using a first feature analysis model that generates the feature quantity of the first weighted input image upon receipt of input of the first weighted input image; generating, by the training apparatus, a feature quantity of the second weighted input image using a second feature analysis model that generates the feature quantity of the second weighted input image upon receipt of input of the second weighted input image; classifying, by the training apparatus, the specimen cell as a benign cell or a malignant cell using a classification model that classifies the specimen cell as a benign cell or a malignant cell upon receipt of input of the feature quantity of the first weighted input image and the feature quantity of the second weighted input image; and updating, by the training apparatus, parameters of the first generative model, the second generative model, the first feature analysis model, the second feature analysis model, and the classification model based on comparison between a classification result and the classification information.

### (Supplementary note 9)

A program for causing a computer to function as a classification apparatus, the program causing the computer to function as: an acquisition means for acquiring a pathological image which includes a specimen cell as a subject; and a classification means, the classification means generating a first weighted input image by processing the pathological image with first weighting information using a first generative model that has been trained to generate, upon receipt of input of the pathological image, the first weighting information for emphasizing a first region of interest in the pathological image, the classification means generating a second weighted input image by processing the pathological image with second weighting information using a second generative model that has been trained to generate, upon receipt of input of the pathological image, the second weighting information for emphasizing a second region of interest, which differs from the first region of interest, in the pathological image, the classification means generating a feature quantity of the first weighted input image using a first feature analysis model that has been trained to generate the feature quantity of the first weighted input image upon receipt of input of the first weighted input image, the classification means generating a feature quantity of the second weighted input image using a second feature analysis model that has been trained to generate the feature quantity of the second weighted input image upon receipt of input of the second weighted input image, and the classification means classifying the specimen cell as a benign cell or a malignant cell using a classification model that has been trained to classify the specimen cell as a benign cell or a malignant cell upon receipt of input of the feature quantity of the first weighted input image and the feature quantity of the second weighted input image.

### (Supplementary note 10)

A program for causing a computer to function as a training apparatus, the program causing the computer to function as: an acquisition means for acquiring (i) a pathological image which includes a specimen cell as a subject and (ii) classification information which indicates whether the specimen cell is a benign cell or a malignant cell; and a training means, the training means generating a first weighted input image by processing the pathological image with first weighting information using a first generative model that generates, upon receipt of input of the pathological image, the first weighting information for emphasizing a first region of interest in the pathological image, the training means generating a second weighted input image by processing the pathological image with second weighting information using a second generative model that generates, upon receipt of input of the pathological image, the second weighting information for emphasizing a second region of interest, which differs from the first region of interest, in the pathological image, the training means generating a feature quantity of the first weighted input image using a first feature analysis model that generates the feature quantity of the first weighted input image upon receipt of input of the first weighted input image, the training means generating a feature quantity of the second weighted input image using a second feature analysis model that generates the feature quantity of the second weighted input image upon receipt of input of the second weighted input image, the training means classifying the specimen cell as a benign cell or a malignant cell using a classification model that classifies the specimen cell as a benign cell or a malignant cell upon receipt of input of the feature quantity of the first weighted input image and the feature quantity of the second weighted input image, and the training means updating parameters of the first generative model, the second generative model, the first feature analysis model, the second feature analysis model, and the classification model based on comparison between a classification result and the classification information.

### [Additional remark 3]

Some or all of the foregoing example embodiments can also be described as below.

A classification apparatus, including at least one processor, the at least one processor carrying out: an acquisition process of acquiring a pathological image which includes a specimen cell as a subject; and a classification process of (i) generating a first weighted input image by processing the pathological image with first weighting information using a first generative model that has been trained to generate, upon receipt of input of the pathological image, the first weighting information for emphasizing a first region of interest in the pathological image, (ii) generating a second weighted input image by processing the pathological image with second weighting information using a second generative model that has been trained to generate, upon receipt of input of the pathological image, the second weighting information for emphasizing a second region of interest, which differs from the first region of interest, in the pathological image, (iii) generating a feature quantity of the first weighted input image using a first feature analysis model that has been trained to generate the feature quantity of the first weighted input image upon receipt of input of the first weighted input image, (iv) generating a feature quantity of the second weighted input image using a second feature analysis model that has been trained to generate the feature quantity of the second weighted input image upon receipt of input of the second weighted input image, and (v) classifying the specimen cell as a benign cell or a malignant cell using a classification model that has been trained to classify the specimen cell as a benign cell or a malignant cell upon receipt of input of the feature quantity of the first weighted input image and the feature quantity of the second weighted input image.

Note that the classification apparatus may further include a memory. The memory may store a program for causing the at least one processor to carry out the acquisition process and the classification process. The program may be stored in a computer-readable non-transitory tangible storage medium.

A training apparatus, including at least one processor, the at least one processor carrying out: an acquisition process of acquiring (i) a pathological image which includes a specimen cell as a subject and (ii) classification information which indicates whether the specimen cell is a benign cell or a malignant cell; and a training process of (i) generating a first weighted input image by processing the pathological image with first weighting information using a first generative model that generates, upon receipt of input of the pathological image, the first weighting information for emphasizing a first region of interest in the pathological image, (ii) generating a second weighted input image by processing the pathological image with second weighting information using a second generative model that generates, upon receipt of input of the pathological image, the second weighting information for emphasizing a second region of interest, which differs from the first region of interest, in the pathological image, (iii) generating a feature quantity of the first weighted input image using a first feature analysis model that generates the feature quantity of the first weighted input image upon receipt of input of the first weighted input image, (iv) generating a feature quantity of the second weighted input image using a second feature analysis model that generates the feature quantity of the second weighted input image upon receipt of input of the second weighted input image, (v) classifying the specimen cell as a benign cell or a malignant cell using a classification model that classifies the specimen cell as a benign cell or a malignant cell upon receipt of input of the feature quantity of the first weighted input image and the feature quantity of the second weighted input image, and (vi) updating parameters of the first generative model, the second generative model, the first feature analysis model, the second feature analysis model, and the classification model based on comparison between a classification result and the classification information.

Note that the training apparatus may further include a memory. The memory may store a program for causing the at least one processor to carry out the acquisition process and the training process. The program may be stored in a computer-readable non-transitory tangible storage medium.

### Reference Signs List

1, 1A: Classification apparatus
2: Training apparatus
11, 11A, 21: Acquisition section
110: First acquisition section
120: Second acquisition section
12: Classification section
22: Training section

## Claims

1. A classification apparatus, comprising:
an acquisition means for acquiring a pathological image which includes a specimen cell as a subject; and
a classification means,
the classification means generating a first weighted input image by processing the pathological image with first weighting information using a first generative model that has been trained to generate, upon receipt of input of the pathological image, the first weighting information for emphasizing a first region of interest in the pathological image,
the classification means generating a second weighted input image by processing the pathological image with second weighting information using a second generative model that has been trained to generate, upon receipt of input of the pathological image, the second weighting information for emphasizing a second region of interest, which differs from the first region of interest, in the pathological image,
the classification means generating a feature quantity of the first weighted input image using a first feature analysis model that has been trained to generate the feature quantity of the first weighted input image upon receipt of input of the first weighted input image,
the classification means generating a feature quantity of the second weighted input image using a second feature analysis model that has been trained to generate the feature quantity of the second weighted input image upon receipt of input of the second weighted input image, and
the classification means classifying the specimen cell as a benign cell or a malignant cell using a classification model that has been trained to classify the specimen cell as a benign cell or a malignant cell upon receipt of input of the feature quantity of the first weighted input image and the feature quantity of the second weighted input image.

2. The classification apparatus according to claim 1, wherein:
at least cytoplasm of the specimen cell is stained; and
the first region of interest and the second region of interest are different in staining intensity from each other.

3. The classification apparatus according to claim 2, wherein:
the second region of interest is a region including stained cilia, and the first region of interest is a region having a staining intensity higher than that of the second region of interest.

4. A training apparatus, comprising:
an acquisition means for acquiring (i) a pathological image which includes a specimen cell as a subject and (ii) classification information which indicates whether the specimen cell is a benign cell or a malignant cell; and
a training means,
the training means generating a first weighted input image by processing the pathological image with first weighting information using a first generative model that generates, upon receipt of input of the pathological image, the first weighting information for emphasizing a first region of interest in the pathological image,
the training means generating a second weighted input image by processing the pathological image with second weighting information using a second generative model that generates, upon receipt of input of the pathological image, the second weighting information for emphasizing a second region of interest, which differs from the first region of interest, in the pathological image,
the training means generating a feature quantity of the first weighted input image using a first feature analysis model that generates the feature quantity of the first weighted input image upon receipt of input of the first weighted input image,
the training means generating a feature quantity of the second weighted input image using a second feature analysis model that generates the feature quantity of the second weighted input image upon receipt of input of the second weighted input image,
the training means classifying the specimen cell as a benign cell or a malignant cell using a classification model that classifies the specimen cell as a benign cell or a malignant cell upon receipt of input of the feature quantity of the first weighted input image and the feature quantity of the second weighted input image, and
the training means updating parameters of the first generative model, the second generative model, the first feature analysis model, the second feature analysis model, and the classification model based on comparison between a classification result and the classification information.

5. The training apparatus according to claim 4, wherein:
at least cytoplasm of the specimen cell is stained; and
the training means sets initial values of the first weighting information and the second weighting information so that the first region of interest and the second region of interest are different in staining intensity from each other.

6. The training apparatus according to claim 5, wherein:
the second region of interest is a region including stained cilia, and the first region of interest is a region having a staining intensity higher than that of the second region of interest; and
the training means sets the initial values of the first region of interest and the second region of interest in the first weighting information and the second weighting information to be relatively higher than those of the other regions.

7. A classification method, comprising:
acquiring, by a classification apparatus, a pathological image which includes a specimen cell as a subject;
generating, by the classification apparatus, a first weighted input image by processing the pathological image with first weighting information using a first generative model that has been trained to generate, upon receipt of input of the pathological image, the first weighting information for emphasizing a first region of interest in the pathological image;
generating, by the classification apparatus, a second weighted input image by processing the pathological image with second weighting information using a second generative model that has been trained to generate, upon receipt of input of the pathological image, the second weighting information for emphasizing a second region of interest, which differs from the first region of interest, in the pathological image;
generating, by the classification apparatus, a feature quantity of the first weighted input image using a first feature analysis model that has been trained to generate the feature quantity of the first weighted input image upon receipt of input of the first weighted input image;
generating, by the classification apparatus, a feature quantity of the second weighted input image using a second feature analysis model that has been trained to generate the feature quantity of the second weighted input image upon receipt of input of the second weighted input image; and
classifying, by the classification apparatus, the specimen cell as a benign cell or a malignant cell using a classification model that has been trained to classify the specimen cell as a benign cell or a malignant cell upon receipt of input of the feature quantity of the first weighted input image and the feature quantity of the second weighted input image.

8. A training method, comprising:
acquiring, by a training apparatus, (i) a pathological image which includes a specimen cell as a subject and (ii) classification information which indicates whether the specimen cell is a benign cell or a malignant cell;
generating, by the training apparatus, a first weighted input image by processing the pathological image with first weighting information using a first generative model that generates, upon receipt of input of the pathological image, the first weighting information for emphasizing a first region of interest in the pathological image;
generating, by the training apparatus, a second weighted input image by processing the pathological image with second weighting information using a second generative model that generates, upon receipt of input of the pathological image, the second weighting information for emphasizing a second region of interest, which differs from the first region of interest, in the pathological image;
generating, by the training apparatus, a feature quantity of the first weighted input image using a first feature analysis model that generates the feature quantity of the first weighted input image upon receipt of input of the first weighted input image;
generating, by the training apparatus, a feature quantity of the second weighted input image using a second feature analysis model that generates the feature quantity of the second weighted input image upon receipt of input of the second weighted input image;
classifying, by the training apparatus, the specimen cell as a benign cell or a malignant cell using a classification model that classifies the specimen cell as a benign cell or a malignant cell upon receipt of input of the feature quantity of the first weighted input image and the feature quantity of the second weighted input image; and
updating, by the training apparatus, parameters of the first generative model, the second generative model, the first feature analysis model, the second feature analysis model, and the classification model based on comparison between a classification result and the classification information.

9. A program for causing a computer to function as a classification apparatus, the program causing the computer to function as:
an acquisition means for acquiring a pathological image which includes a specimen cell as a subject; and
a classification means,
the classification means generating a first weighted input image by processing the pathological image with first weighting information using a first generative model that has been trained to generate, upon receipt of input of the pathological image, the first weighting information for emphasizing a first region of interest in the pathological image,
the classification means generating a second weighted input image by processing the pathological image with second weighting information using a second generative model that has been trained to generate, upon receipt of input of the pathological image, the second weighting information for emphasizing a second region of interest, which differs from the first region of interest, in the pathological image,
the classification means generating a feature quantity of the first weighted input image using a first feature analysis model that has been trained to generate the feature quantity of the first weighted input image upon receipt of input of the first weighted input image,
the classification means generating a feature quantity of the second weighted input image using a second feature analysis model that has been trained to generate the feature quantity of the second weighted input image upon receipt of input of the second weighted input image, and
the classification means classifying the specimen cell as a benign cell or a malignant cell using a classification model that has been trained to classify the specimen cell as a benign cell or a malignant cell upon receipt of input of the feature quantity of the first weighted input image and the feature quantity of the second weighted input image.

10. A program for causing a computer to function as a training apparatus, the program causing the computer to function as:
an acquisition means for acquiring (i) a pathological image which includes a specimen cell as a subject and (ii) classification information which indicates whether the specimen cell is a benign cell or a malignant cell; and
a training means,
the training means generating a first weighted input image by processing the pathological image with first weighting information using a first generative model that generates, upon receipt of input of the pathological image, the first weighting information for emphasizing a first region of interest in the pathological image,
the training means generating a second weighted input image by processing the pathological image with second weighting information using a second generative model that generates, upon receipt of input of the pathological image, the second weighting information for emphasizing a second region of interest, which differs from the first region of interest, in the pathological image,
the training means generating a feature quantity of the first weighted input image using a first feature analysis model that generates the feature quantity of the first weighted input image upon receipt of input of the first weighted input image,
the training means generating a feature quantity of the second weighted input image using a second feature analysis model that generates the feature quantity of the second weighted input image upon receipt of input of the second weighted input image,
the training means classifying the specimen cell as a benign cell or a malignant cell using a classification model that classifies the specimen cell as a benign cell or a malignant cell upon receipt of input of the feature quantity of the first weighted input image and the feature quantity of the second weighted input image, and
the training means updating parameters of the first generative model, the second generative model, the first feature analysis model, the second feature analysis model, and the classification model based on comparison between a classification result and the classification information.
